# EUROPEAN PATENT APPLICATION

(11) **EP 0 982 027 A1**
(43) Date of publication of application: **01.03.2000**
(21) Application number: 98115401.6
(22) Date of filing: 17.08.1998
(51) Int. Cl.: A61K 31/335

(54) **Taxoid derivatives for treating abnormal cell proliferation of the brain**

(71) Applicant: RHONE-POULENC RORER S.A., 92160 Antony (FR)
(72) Inventor: Bissery, Marie- Christie, 94400 Vitry sur Seine (FR); Vrignaud, Patricia, 77380 Combs la Ville (FR); Roberts, Simon, Harlow, Essex, CM17 9PG (GB); Brealey, Clive, Wigginton, Tring Herts HP23 6HE (GB)
(74) Representative: Le Pennec, Magali

(57) **Abstract**

The present invention relates a new use of taxoid derivatives. It relates more precisely a method for treating abnormal cell proliferation in the brain of mammals including men by administrating a taxoid derivative.

## Description

The present invention relates a new use of taxoid derivatives. It relates more precisely a method for treating abnormal cell proliferation in the brain of mammals including men by administrating a compound of general formula (I) or a pharmaceutically acceptable salt or solvante thereof: 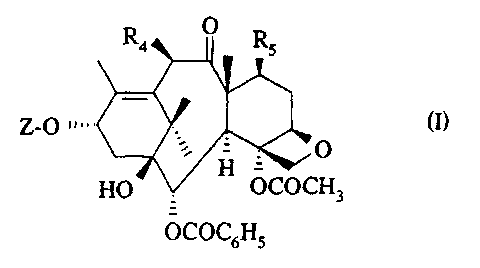 in which:
Z represents a hydrogen atom or a radical of general formula: 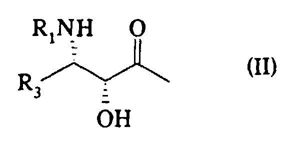 in which:
R₁ represents
a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals,
a thenoyl or furoyl radical or
a radical R₂-O-CO- in which R₂ represents:
   - an alkyl radical containing 1 to 8 carbon atoms,
   - an alkenyl radical containing 2 to 8 carbon atoms,
   - an alkynyl radical containing 3 to 8 carbon atoms,
   - a cycloalkyl radical containing 3 to 6 carbon atoms,
   - a cycloalkenyl radical containing 4 to 6 carbon atoms or
   - a bicycloalkyl radical containing 7 to 10 carbon atoms,
      these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
   - a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or
   - a 5-membered aromatic heterocyclic radical preferably chosen from furyl and thienyl radicals,
   - or a saturated heterocyclic radical containing 4 to 6 carbon atoms, optionally substituted with one or more alkyl radicals containing 1 to 4 carbon atoms,
R₃ represents
an unbranched or branched alkyl radical containing 1 to 8 carbon atoms,
an unbranched or branched alkenyl radical containing 2 to 8 carbon atoms,
an unbranched or branched alkynyl radical containing 2 to 8 carbon atoms,
a cycloalkyl radical containing 3 to 6 carbon atoms,
a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals,
on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals,
R₄ represents
an alkoxy radical containing 1 to 6 carbon atoms in an unbranched or branched chain,
an alkenyloxy radical containing 3 to 6 carbon atoms in an unbranched or branched chain,
an alkynyloxy radical containing 3 to 6 carbon atoms in an unbranched or branched chain,
a cycloalkyloxy radical containing 3 to 6 carbon atoms or
a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
R₅ represents
an alkoxy radical containing 1 to 6 carbon atoms in an unbranched or branched chain,
an alkenyloxy radical containing 3 to 6 carbon atoms,
an alkynyloxy radical containing 3 to 6 carbon atoms,
a cycloalkyloxy radical containing 3 to 6 carbon atoms or
a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

Preferably, the aryl radicals which can be represented by R₃ are phenyl or α- or β-naphthyl radicals optionally substituted with one or more atoms or radicals chosen from halogen atoms (fluorine, chlorine, bromine, iodine) and alkyl, alkenyl, alkynyl, aryl, arylalkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, dialkylcarbamoyl, cyano, nitro and trifluoromethyl radicals, on the understanding that the alkyl radicals and the alkyl portions of the other radicals contain 1 to 4 carbon atoms, that the alkenyl and alkynyl radicals contain 2 to 8 carbon atoms and that the aryl radicals are phenyl or α- or β-naphthyl radicals.

Preferably, the heterocyclic radicals which can be represented by R₃ are 5-membered aromatic heterocyclic radicals containing one or more identical or different atoms chosen from nitrogen, oxygen and sulphur atoms, optionally substituted with one or more identical or different substituents chosen from halogen atoms (fluorine, chlorine, bromine, iodine) and alkyl radicals containing 1 to 4 carbon atoms, aryl radicals containing 6 to 10 carbon atoms, alkoxy radicals containing 1 to 4 carbon atoms, aryloxy radicals containing 6 to 10 carbon atoms, amino radicals, alkylamino radicals containing 1 to 4 carbon atoms, dialkylamino radicals in which each alkyl portion contains 1 to 4 carbon atoms, acylamino radicals in which the acyl portion contains 1 to 4 carbon atoms, alkoxycarbonylamino radicals containing 1 to 4 carbon atoms, acyl radicals containing 1 to 4 carbon atoms, arylcarbonyl radicals in which the aryl portion contains 6 to 10 carbon atoms, cyano, carboxyl or carbamoyl radicals, alkylcarbamoyl radicals in which the alkyl portion contains 1 to 4 carbon atoms, dialkylcarbamoyl radicals in which each alkyl portion contains 1 to 4 carbon atoms or alkoxycarbonyl radicals in which the alkoxy portion contains 1 to 4 carbon atoms.

Preferably, the radicals R₄ and R₅, which may be identical or different, represent unbranched or branched alkoxy radicals containing 1 to 6 carbon atoms, optionally substituted with a methoxy, ethoxy, ethylthio, carboxyl, methoxycarbonyl, ethoxycarbonyl, cyano, carbamoyl, N-methylcarbamoyl, N-ethylcarbamoyl, N,N-dimethylcarbamoyl, N,N-diethylcarbamoyl, N-pyrrolidinocarbonyl or N-piperidinocarbonyl radical.

More specially, the present invention relates to the products of general formula (I) in which Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl (methyl), alkoxy (methoxy), dialkylamino (dimethylamino), acylamino (acetylamino), alkoxycarbonylamino (tert-butoxycarbonylamino) or trifluoromethyl radicals, or a 2- or 3-furyl, 2- or 3-thienyl or 2-, 4- or 5-thiazolyl radical, and R₄ and R₅, which may be identical or different, each represent an unbranched or branched alkoxy radical containing 1 to 6 carbon atoms.

Still more specially, the present invention relates to the products of general formula (I) in which Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R₄ and R₅, which may be identical or different, each represent a methoxy, ethoxy or propoxy radical.

Of even more special interest are the products of general formula (I) in which R₃ represents a phenyl radical and R₁ represents a ter-butoxycarbonyl radical, R₄ and R₅, which may be identical or different, represent a methoxy, ethoxy or propoxy radical.

In a still higher interest, the present invention relates to 4α-acetoxy-2α-benzoyloxy-5β,20-epoxy-1β-hydroxy-7β,10β-dimethoxy-9-oxo-11-taxen-13α-yl (2R,3S)-3 tert-butoxycarbonylamino-2-hydroxy-3-phenylpropionate of formula (Ia)

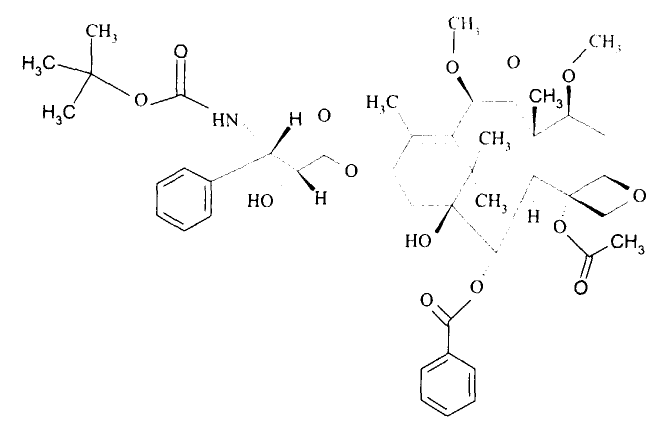

It is known, from patent WO 96/30355, to prepare a derivative according to the present invention by two processes. According to a first, multi-step process, starting with 10-deacetylbaccatin III of formula: 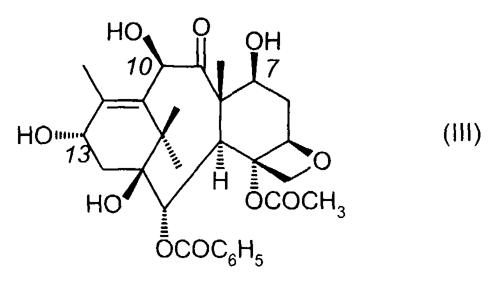 it is selectively protected in positions 7 and 13, for example in the form of a silyl diether, followed by the action of a product of general formula:

R-X (IV)

in which R represents a radical as defined above and X represents a reactive ester residue such as a sulphuric or sulphonic ester residue or a halogen atom, to give a product bearing the unit -OR in position 10 and silyl groups in positions 7 and 13. Next, the silyl protecting groups are replaced with hydrogen atoms to give a compound still bearing the group -OR in position 10 and OH groups in positions 7 and 13. The latter derivative is etherified selectively in position 7 by reaction with the derivative of formula IV to give the derivative of formula (I) in which Z is equal to hydrogen.

The final step consists in esterifying in position 13, according to a process which is known per se, the derivatives of formula (Ia), in which Z represents hydrogen, in the presence of a β-lactam according, for example, to the process described in patent EP 617,018, or in the presence of an oxazolidine as described, for example, in patent WO 96/30355 mentioned above. After deprotection of the protecting groups in positions 7 and 10, an ester of formula (Ia) is thus obtained in which Z is other than hydrogen and R represents hydrogen. The next step consists in reacting the positions 7 and 10 simultaneously by the action of a reagent formed in situ from a sulphoxide of formula (V) and acetic anhydride (Pummerer-type reaction),

R-SO-R (V)

in which R has the same meaning as above, to form an alkylthioalkyloxy-type intermediate on positions 7 and 10.

The final step, which allows the desired compound of formula (Ia) to be obtained, is carried out on the intermediate compound obtained above, by the action of activated Raney nickel.

Generally, the action of the reagent formed in situ from sulphoxide of general formula (V), preferably dimethyl sulphoxide and acetic anhydride, is carried out in the presence of acetic acid or an acetic acid derivative such as a haloacetic acid, at a temperature of between 0 and 50°C.

Generally, the action of the activated Raney nickel in the presence of an aliphatic alcohol or an ether is carried out at a temperature of between -10 and 60°C.

In the FR 97-14442 application a further process has been described. This invention allows, in a single step, the direct, selective and simultaneous alkylation of the two hydroxyl functions in positions 7 and 10 of 10-deacetylbaccatin or of derivatives thereof esterified in position 13, of formula (VI) 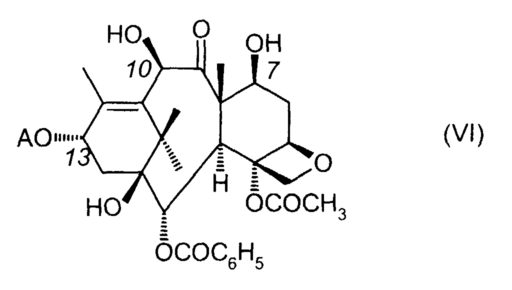 in which A represents hydrogen or a side chain of formula (IIa) below: 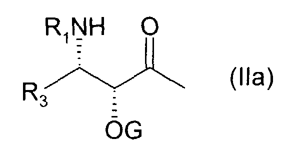 in which G represents a protecting group for the hydroxyl function, R₁ and R₃ have the same meaning as in formula (II)
or an oxazolidine unit of formula (Id): 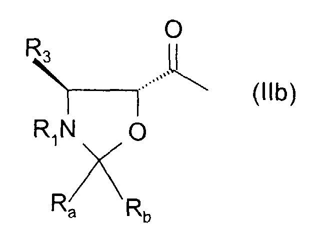 in which R₁ and R₃ have the same meaning as in formula (II), Rₐ and R_{b}, which may be identical or different, represent hydrogen or alkyl, aryl, halo, alkoxy, arylalkyl, alkoxyaryl, haloalkyl, haloaryl, it being possible for the substituents optionally to form a 4- to 7-membered ring.

It is preferred to use 10-deacetylbaccatin as starting material, i.e. the product of formula (III), which allows appreciable economy as regards the process and moreover avoids the intermediate protection and deprotection steps necessary in the old processes.

Among the groups G for protecting the hydroxyl function of formula (IIa), it is generally preferred to choose all of the protecting groups described in books such as Greene and Wuts, Protective Groups in Organic Synthesis, 1991, John Wiley & Sons, and MacOmie, Protective Groups in Organic Chemistry, 1975, Plenum Press, and which are deprotected under conditions which degrade the rest of the molecule little or not at all, such as, for example:
- ethers, and preferably ethers such as methoxymethyl ether, 1-ethoxyethyl ether, benzyloxymethyl ether, p-methoxybenzyloxymethyl ether, benzyl ethers optionally substituted with one or more groups such as methoxy, chloro, nitro, 1-methyl-1-methoxyethyl ether, 2-(trimethylsilyl)ethoxymethyl ether, tetrahydropyranyl ether and silyl ethers such as trialkylsilyl ethers,
- carbonates such as trichloroethyl carbonates.

More particularly, the radicals Rₐ and R_{b} of general formula (IIb) are chosen from those described in patent WO 94/07878 and the derivatives more particularly preferred are those in which Rₐ is hydrogen and R_{b} is a p-methoxyphenyl radical.

The alkylating agent is chosen from:
- alkyl halides, and preferably from alkyl iodides (RI)
- alkyl sulphates such as methyl sulphate
- oxoniums such as trialkyloxonium boric salts, in particular trimethyloxonium tetrafluoroborate (Me₃OBF₄).

Methyl iodide is preferably used.

The alkylating agent is used in the presence of an anionization agent such as one or more strong bases, in anhydrous medium.

Among the bases which can be used in anhydrous medium, mention may be made of:
- alkali metal hydrides such as sodium or potassium hydride
- alkali metal alkoxides such as potassium tert-butoxide
- silver oxide Ag₂O
- 1,8-bis(dimethylamino)naphthalene
- mono- or dimetallic base mixtures such as those described, for example, in publications such as P. Caubère Chem. Rev. 1993, 93, 2317-2334 or M. Schlosser Mod. Synth. Methods (1992), 6, 227-271; in particular the alkyllithium/alkali metal t-butoxide or alkali metal amide/alkali metal t-butoxide combinations are preferred. One of the two bases can be generated "in situ".

Among all of the possible combinations of alkylating agent and anionization agent, it is preferred to use methyl iodide in the presence of potassium hydride.

The reaction is preferably carried out in an organic medium which is inert under the reaction conditions. Among the solvents, it is preferred to use:
- ethers such as tetrahydrofuran or dimethoxyethane
- when silver oxide is used, it is preferred to use polar aprotic solvents such as dimethylformamide, or aromatic solvents such as toluene
- when 1,8-bis(dimethylamino)naphthalene is used, it is preferred to use alkylesters such as ethylacetate.

For better implementation of the invention, it is preferred to use a molar ratio between the anionization agent and the substrate of greater than 2 and preferably between 2 and 20.

It is also preferred to use a molar ratio between the alkylating agent and the substrate of greater than 2 and preferably between 2 and 40.

It is preferred to use a reaction temperature of between -30°C and 80°C.

The reaction time advantageously ranges between a few hours and 48 hours depending on the reagents chosen.

After the alkylating step, when the latter is carried out on 10-deacetylbaccatin, the process then proceeds, in a known manner, to the esterification step according, for example, to the processes described in patents EP 617,018 or WO 96/30355 mentioned above.

Thus, according to a first, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with a suitably protected β-lactam in the presence of an activating agent chosen from tertiary amines and metal bases which ensure the formation of an alkoxide in position 13. Deprotection of the side chain is then achieved by the action of an inorganic or organic acid.

Thus, according to a second, 3-step process, the procedure first begins with the dialkylation of 10-deacetylbaccatin, using an alkylating agent in the presence of a strong base, in a second step, the 10-deacetylbaccatin dietherified in positions 7 and 10 is coupled, in position 13, with an oxazolidine in the presence of a coupling agent such as diimides in the presence of an activating agent such as dialkylaminopyridines. Opening of the oxazolidine is achieved by the action of an inorganic or organic acid.

According to a third process, the procedure begins with the esterification in position 13 of baccatin suitably protected in positions 7 and 10, with a β-lactam or an oxazolidine in the presence of a coupling agent and/or an activating agent as described in the above two processes. After deprotection in positions 7 and 10, the dietherification in positions 7 and 10 is carried out by an alkylating agent in the presence of a strong base. Deprotection of the side chain is then achieved by the action of an inorganic or organic acid.

The products of general formula (I) have remarkable biological properties.

In vitro, measurement of the biological activity is carried out on tubulin extracted from pig brain by the method of M.L. Shelanski et al., Proc. Natl. Acad. Sci. USA, 70, 765-768 (1973). The study of the depolymerization of the microtubules into tubulin is carried out according to the method of G. Chauvière et al., C.R. Acad. Sci., 293, série II, 501-503 (1981).

In vivo, the products of general formula (I) proved active in mice grafted with the B16 melanoma at doses of berween 1 and 50 mg/kg intraperitoneally, as well as on other liquid or solid tumours.

The compounds have anti-tumor properties, more particularly, activity against tumors which are resistant to Taxol® and Taxotere®. Such tumors include, for example, brain tumors which have an elevated expression of mdr 1 gene (multi-drug resistant gene). Multi-drug resistance is the usual term relating to the resistance by a tumor against various compounds having differing structures and mechanisms of action. Taxoids are generally known to be highly recognized by experimental tumors such as P388/DOX, a P388 murine leukemia cell line selected for doxorubicin (DOX) resistance, which express mdr 1. The compounds according to the present invention are less recognized by P388/DOX. More particularly, the compounds are less recognized than Taxotere® by mdr 1.

The compounds of formula (I) are mainly used for preparing a medecine for treating abnormal cell proliferation in the brain.

The compound and mainly compound fo formula (I) where R₄ and R₅ are each methoxy has the property to cross the blood brain barrier. It is active compared to the other known taxoids such as Taxol® or Taxotere® to treat the brain cancer.

The product of formula (I) can be used concurrently with at least other therpaeutic treatment. It is more preferably used with other therapeutic treatment comprising antineoplastic drugs, monoclonal antibodies, immunotherapies, radiotherapies, or biological response modifiers. Among the biological responses modifier lymphokines and cytokines, interleukins, α, β, or δ interfeons and TNF are preferably used.

The product of formula (I) is preferably administered by parenteral administration such as intravenous, intraperitoneal, intramuscular or subcutaneous administration.

### Example

### 1. INTRODUCTION

Product of formula (Ia) is a potent anti-cancer agent in pre-clinical models. Reported here are the analytical results obtained from a single i.v. bolus pharmacokinetic study in the mouse.

Groups of female C3H/HeN mice received the product by the intravenous route as a bolus at a dose level of 40 mg.kg⁻¹ equivalent to 120 mg.m⁻². Blood and brain samples were obtained from all dosed animals sacrificed at intervals up to 72 hours post dose. Brain and corresponding plasma samples have been assayed for product Ia content by an LC-MS/MS assay.

### 2. METHODS

Formulation: 2.25 mg.ml⁻¹ solution containing 5 % Polysorbate 80, 5 % ethanol and 90 % of an aqueous 5 % glucose solution.

Fifty-six female C3H/HeN mice each weighing ca 20 g were each administered formulated product II by i.v. bolus via the tail vein at an injection volume of 0.4 ml to give a total dose of 40 mg.kg⁻¹.

### Blood and Tissue Sampling

Sampling : Blood by cardiac puncture and liver and brain by dissection after CO₂ sacrifice
Sample Times : at 2, 5, 15, 30, 45 minutes, 1, 2, 4, 6, 8, 14, 24, 48 and 72 hours post dose.

Whole blood was collected into heparinised tubes and corresponding plasma samples were obtained by centrifugation and frozen immediately at -20°C. Tissues were blotted, weighed and frozen immediately at -20°C. All samples were dispatched frozen for analysis. Upon receipt, samples were stored frozen at approximately -18°C pending analysis.

### LC-MS/MS Analysis of Brain and Plasma Samples

Detection: LC-MS/MS (Sciex API III plus) in turbo-ionspray mode

The following MS conditions were applied:

| | | |
|---|---|---|
| Auxiliary gas flow | 6 L.min⁻¹ | |
| Nebuliser gas flow | 0.6 L.min⁻¹ | |
| Turbo temperature | 450°C | |
| CGT | 300 | Curtain gas |
| flow 0.6 L.min⁻¹ | Scan time | 1 |
| scan/sec | Eluent split ratio | 1:10 |

- Column:: 75 x 4.6 mm Supelcosil ABZ plus (3 µm).
- Mobile phase:: Acetonitrile/methanol/ammonium acetate (10mM) ; 40/25/35 v/v/v.
- Flow rate :: 1 ml.min⁻¹.
- Temperature :: Ambient.
- Extraction :: Plasma: Add 100 µl of acetonitrile to sample (50 µl). Vortex, centrifuge, remove supernatant, add 100 µl of mobile phase and inject 150 µl.
Brain : Add 100 µl of acetonitrile to homogenised sample (100 mg of a 1:1 w/w brain with water homogenate) and vortex. Add 1ml of diethyl ether, vortex, centrifuge, remove organic layer and dry under N₂. Reconstitute in 200 µl of mobile phase and inject 150 µl.
- Calibration standards:: *Plasma* : Nine at concentrations of 5, 10, 20, 50, 100, 200, 300, 400 & 500 ng.ml⁻¹ (product Ia). Prepared by adding suitable aliquots of the product (concentrations = 0.1, 1 or 10 µg.ml⁻¹) in ethanol to 0.5 ml aliquots of mouse plasma. Each sample was vortexed following drug addition; a 50 µl aliquot was then removed for assay.
*Brain* : Eleven at concentrations of 10, 20, 30, 100, 200, 300, 400, 500, 1000, 2500 & 5000 ng.g⁻¹ in homogenised mouse brain (1:1 w/w brain with water). Prepared by adding suitable aliquots of the product (concentrations = 1, 10 or 100 µg.ml⁻¹) in ethanol to 0.5, 1, 3 or 4 g of homogenised mouse brain. Each sample was vortexed following drug addition and a 100 mg aliquot was then removed for assay.
- Retention times:: Drug ; product Ia: ∼2.3 min.
- Extraction efficiency :: *Plasma* : ca.58 % at 200 ng.ml⁻¹.
*Brain* : ca.41 % at 500 ng.g⁻¹ and 39 % at 1000 ng.g⁻¹.

### 3. RESULTS

### 3.1 Plasma Levels

The following table contains product Ia plasma levels observed after i.v. administration of product Ic at a dose level of 40 mg.kg⁻¹ to the mouse.

**Table 1**

| Preliminary plasma concentrations of product Ia after i.v. dosing at a level of 40 mg.kg⁻¹ to the mouse | | | | | | |
|---|---|---|---|---|---|---|
| Time after dose (h) | Plasma product Ic concentration (ng.ml⁻¹) | | | | | |
| | IV1 | IV2 | IV3 | IV4 | MEAN | ± s.d. |
| 2 min | 52987 | 45942 | 49607 | 38994 | 46882 | 5994 |
| 5 min | 36734 | 33538 | 32077 | 34903 | 34313 | 1984 |
| 15 min | 20493 | 20897 | 21051 | 19459 | 20475 | 717 |
| 0.5h | 10765 | 10344 | 9170 | 11232 | 10378 | 883 |
| 0.75h | 7133 | 10948 | 8121 | 10148 | 9087 | 1764 |
| 1h | 6017 | 7423 | 6693 | 6079 | 6553 | 655 |
| - | 4633 | 4337 | 4600 | 3564 | 4283 | 498 |
| 4h | 1072 | 1110 | 835 | 830 | 962 | 150 |
| 6h | 449 | 316 | 346 | 336 | 362 | 59 |
| 8h | 204 | 199 | 195 | 154 | 188 | 23 |
| 14h | 65 | 56 | 50 | 52 | 56 | 7 |
| 24 | 18(blq) | 15(blq) | 15(blq) | 16(blq) | 16 | 1 |
| 48 | 4(blq) | n.d. | n.d. | 4(blq) | 2 | 2 |
| 72 | n.d. | n.d. | n.d. | n.d. | --- | n.a. |
| n.a.: not applicable n.d.: not detected (≤ l.o.d. of 4 ng.ml⁻¹) blq: below limit of accurate quantification (20 ng.ml⁻¹). | | | | | | |

### 3.2 Brain Levels

The following table contains product Ia whole brain levels observed after i.v. administration of product Ia at a dose level of 40 mg.kg⁻¹ to the mouse.

**Table 2**

| Preliminary brain concentration for product Ic after i.v. dosing at a level of 40 mg.kg⁻¹ to the mouse | | | | | | |
|---|---|---|---|---|---|---|
| Time after dose (h) | Brain product Ic concentration (ng.g⁻¹) | | | | | |
| | IV1 | IV2 | IV3 | IV4 | MEAN | ± s.d. |
| 2 min | 6962 | 8817 | 8147 | 7630 | 7889 | 786 |
| 5 min | 8344 | 8473 | 7762 | 8091 | 8167 | 313 |
| 15 min | 5809 | 7100 | 7641 | 6481 | 6758 | 791 |
| 0.5h | 7262 | 6788 | 8317 | 6894 | 7315 | 698 |
| 0.75h | 7675 | 8086 | 7513 | 7272 | 7637 | 342 |
| 1h | 6424 | 8964 | 1747 | 7489 | 6156 | 3118 |
| 2h | 7956 | 8418 | 6966 | 7017 | 7589 | 716 |
| 4h | 7909 | 6939 | 6712 | 5459 | 6755 | 1008 |
| 6h | 6688 | 7968 | 7350 | 3712 | 6430 | 1886 |
| 8h | 9067 | 6977 | 8616 | 8342 | 8250 | 900 |
| 14h | 9618 | 10049 | 7595 | 9271 | 9133 | 1074 |
| 24 | 7905 | 9842 | 7885 | 9052 | 8671 | 952 |
| 48 | 6660 | 8541 | 7704 | 7986 | 7723 | 789 |
| 72 | 5899 | 5511 | 5692 | 3894 | 5249 | 917 |
| n.d.: not detected (<l.o.d. of 92 ng.g⁻¹) n.a.: not applicable | | | | | | |

### 3.3 Pharmacokinetic Parameters

The following table contains the preliminary pharmacokinetic parameters for product Ic derived after i.v. administration to the mouse at 40 mg.kg⁻¹ calculated using mean plasma and brain level data.

**Table 3**

| Preliminary mean pharmacokinetic data | | | | | |
|---|---|---|---|---|---|
| Sample | AUC_{0-∞} (h.µg.ml⁻¹ or .g⁻¹) | Cl_{T} (l.h⁻¹.kg⁻¹) | Vdss (l.kg⁻¹) | Initial T_{1/2} (h) | Terminal T_{1/2} (h) |
| Plasma+ | 30.0 | 1.3 | 1.9 | 0.7 | 6.2 |
| Plasma# | 29.8 | 1.3 | 2.4 | 0.2 | 2.0 |
| Brain | 787.8* | n.a. | n.a. | --- | 31.4 |

| | | | | | |
|---|---|---|---|---|---|
| + calculated from values ≥ l.o.d. of 4 ng.ml⁻¹ | | | | | |
| # calculated from values ≥ b.l.q. of 20 ng.ml⁻¹ | | | | | |
| * the corresponding AUC₀₋₇₂ₕ = 549.7 h.µg.g⁻¹ | | | | | |

A biexponential equation was fitted to the profiles using an interactive linear least-square algorithm as part of the SIPHAR package. AUC was calculated by the trapezoidal rule from time 0 to both the time of the last value that was equal to or greater than the l.o.d.⁺ (4 ng.ml⁻¹) or the l.o.q.^{#} (20 ng.ml⁻¹) for plasma and up to 72h post dose for the brain, and then extrapolated to infinity.

### Key:

- AUC_{0-∞}:: Area under the plasma or brain concentration versus time curve from t=0 (start of infusion) to infinity.
- Initial T_{1/2}:: Initial (distribution) half-life.
- Terminal T_{1/2}:: Terminal (elimination) half-life (should be regarded as an estimate only being dependent on sampling frequency in the terminal phase and assay sensitivity).
- Cl_{T}:: Total plasma clearance.
- Vdss:: Volume of distribution at steady state.
- n.a.:: Not applicable.

### 4. CONCLUSIONS

- Product Ic levels were high as would be expected after an i.v. dose of 40 mg.kg⁻¹ but declined rapidly from the peak at 2 minutes (mean of 46.9 µg.ml⁻¹) to less than 1 µg.ml⁻¹ within 4h (initial half-life of ≤ 0.7h). However levels persisted above the limit of accurate quantification (20 ng.ml⁻¹) up to 14h post dose and consistently above the limit of detection (4 ng.ml⁻¹) for up to 24h post dose.
- A terminal half-life of 6.2 h was calculated from detectable plasma levels (≥4 ng.ml⁻¹). However, it should be noted that the terminal half-life is very dependent on assay sensitivity in this case and if levels above the limit of accurate quantification (20 ng.ml⁻¹) are utilised to calculate pharmacokinetic parameters instead, then the terminal half-life drops to 2.0h.
   Mean total plasma clearance was determined to be 1.3 l.h⁻¹.kg⁻¹ which represents a significant fraction of average liver plasma flow (based on average liver blood flow of ca 5.2 l.h⁻¹.kg⁻¹).
- In this species post-i.v. administration, product Ic appears to readily penetrate the blood brain barrier. High levels were detected at the first sampling time (7.9 µg.g⁻¹ at 2 mins) indicating rapid uptake into this tissue. Although peak levels of 9.1 µg.g⁻¹ were observed at 14h, high concentrations were sustained up to the last sampling time (5.3 µg.g⁻¹ at 72h). Not surprisingly the product is slowly cleared from the brain with a half-life of 31.4h. On the basis of AUC_{0-∞} values (788 h.µg.g⁻¹ versus 30 h.µg.ml⁻¹), product Ia levels in the brain were about twenty times those in the plasma.

## Claims

1. Use of a compound of formula (I) for preparing a medecine for treating abnormal cell proliferation in the brain, said use comprising administration to a mammal of an effective amount of a compound of formula (I) or a pharmaceutically acceptable salt or solvate. 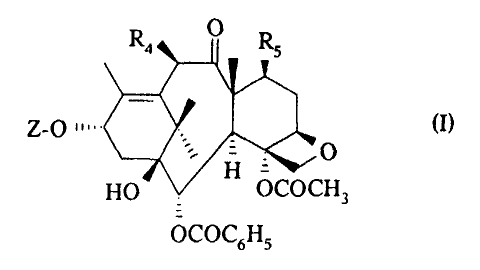 in which:
Z represents a hydrogen atom or a radical of general formula: 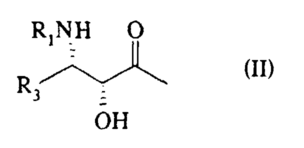 in which:
R₁ represents
a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals,
a thenoyl or furoyl radical or
a radical R₂-O-CO- in which R₂ represents:
an alkyl radical containing 1 to 8 carbon atoms,
an alkenyl radical containing 2 to 8 carbon atoms,
an alkynyl radical containing 3 to 8 carbon atoms,
a cycloalkyl radical containing 3 to 6 carbon atoms,
a cycloalkenyl radical containing 4 to 6 carbon atoms or
a bicycloalkyl radical containing 7 to 10 carbon atoms,
these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,
- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or
- a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,
R₃ represents
an unbranched or branched alkyl radical containing 1 to 8 carbon atoms,
an unbranched or branched alkenyl radical containing 2 to 8 carbon atoms,
an unbranched or branched alkynyl radical containing 2 to 8 carbon atoms,
a cycloalkyl radical containing 3 to 6 carbon atoms,
a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals,
or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals,
on the understanding that, in the substituents of the phenyl, α- or β-naphthyl and aromatic heterocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or I- or ϑ-naphthyl radicals,
R₄ represents
an alkoxy radical containing 1 to 6 carbon atoms in an unbranched or branched chain,
an alkenyloxy radical containing 3 to 6 carbon atoms in an unbranched or branched chain,
an alkynyloxy radical containing 3 to 6 carbon atoms in an unbranched or branched chain,
a cycloalkyloxy radical containing 3 to 6 carbon atoms or
a cycloalkenyloxy radical containing 4 to 6 carbon atoms,
these radicals being optionally substituted with one or more halogen atoms or with an alkoxy radical containing 1 to 4 carbon atoms, an alkylthio radical containing 1 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms,
R₅ represents
an alkoxy radical containing 1 to 6 carbon atoms in an unbranched or branched chain,
an alkenyloxy radical containing 3 to 6 carbon atoms,
an alkynyloxy radical containing 3 to 6 carbon atoms,
a cycloalkyloxy radical containing 3 to 6 carbon atoms or
a cycloalkenyloxy radical containing 3 to 6 carbon atoms,
these radicals being optionally substituted with one or more halogen atoms or with an alkoxy radical containing 1 to 4 carbon atoms, an alkylthio radical containing 2 to 4 carbon atoms or a carboxyl radical, an alkyloxycarbonyl radical in which the alkyl portion contains 1 to 4 carbon atoms, a cyano or carbamoyl radical or an N-alkylcarbamoyl or N,N-dialkylcarbamoyl radical in which each alkyl portion contains 1 to 4 carbon atoms or, with the nitrogen atom to which it is linked, forms a saturated 5- or 6-membered heterocyclic radical optionally containing a second hetero atom chosen from oxygen, sulphur or nitrogen atoms, optionally substituted with an alkyl radical containing 1 to 4 carbon atoms or a phenyl radical or a phenylalkyl radical in which the alkyl portion contains 1 to 4 carbon atoms.

2. Use of compound according to claim 1 wherein Z represents a hydrogen atom or a radical of general formula (II) in which R₁ represents a benzoyl radical or a radical R₂-O-CO- in which R₂ represents a tert-butyl radical and R₃ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms and alkyl, alkoxy, dialkylamino, acylamino, alkoxycarbonylamino or trifluoromethyl radical, or a 2- or 3-furyl, 2- or 3-thienyl or 2-, 4- or 5-thiazolyl radical, and R₄ and R₅, which may be identical or different, each represent an unbranched or branched alkyloxy radical containing 1 to 6 carbon atoms.

3. Use of a compound according to claim 2 wherein Z represents a hydrogen atom or a radical of general formula (II) in which R₁ represents a benzoyl radical or a radical R₂-O-CO- in which R₂ represents a tert-butyl radical and R₃ represents an isobutyl, isobutenyl, butenyl, cyclohexyl, phenyl, 2-furyl, 3-furyl, 2-thienyl, 3-thienyl, 2-thiazolyl, 4-thiazolyl or 5-thiazolyl radical, and R₄ and R₅, which may be identical or different, each represent a methoxy, ethoxy or propoxy radical.

4. Use of 4α-acetoxy-2α-benzoyloxy-5β,20-epoxy-1β-hydroxy-7β,10β-dimethoxy-9-oxo-11-taxen-13α-yl (2R,3S)-3-tert-butoxycarbonylamino-2-hydroxy-3-phenylpropionate. for preparing a medecine for treating abnormal cell proliferation in the brain.

5. Use of compound according to claims 1 to 4 wherein abnormal cell proliferation is cancer of the brain.

6. Use of compound according to claim 5 wherein this use is performed concurrently with at least other therapeutic treatment.

7. Use of compound according to claim 6 wherein the other therapeutic treatment comprises antineoplastic drugs, monoclonal antibodies, immunotherapies, radiotherapies, or biological response modifiers.

8. Use of compound according to claim 7 wherein the response modifiers comprise lymphokines and cytokines.

9. Use of compound according to claim 8 wherein the response modifies comprise interleukins, α, β, or δ interfeons and TNF.

10. Use of compound according to claim 1 wherein the medecine is administered by parenteral administration.

11. Use of compound to claim 10 wherein the compound of formula (I) is administered by intravenous, intraperitoneal, intramuscular or subcutaneous administration.
